# EUROPEAN PATENT SPECIFICATION

(11) **EP 1 458 425 B1**
(45) Date of publication and mention of the grant of the patent: **18.11.2009**
(21) Application number: 02790278.2
(22) Date of filing: 20.12.2002
(51) Int. Cl.: A61L 24/10, A61L 24/04, B01F 13/00, B01F 15/02, B65D 25/08, A61K 9/00, B01F 3/12, A61L 33/12

(54) **A HEMOSTATIC KIT, A METHOD OF PREPARING A HEMOSTATIC AGENT AND A METHOD OF PROMOTING HEMOSTASIS**
HÄMOSTATISCHE GARNITUR, VERFAHREN ZUM ZUBEREITEN EINES HÄMOSTATISCHEN MITTELS UND VERFAHREN ZUR BLUTSTILLUNG
KIT HEMOSTATIQUE, PROCEDE DE PREPARATION D'AGENT HEMOSTATIQUE ET PROCEDE FAVORISANT L'HEMOSTASE

(30) Priority: 21.12.2001 DK 200101941; 27.03.2002 EP 02388028
(43) Date of publication of application: 22.09.2004
(60) Divisional application: 09159082.8
(73) Proprietor: Ferrosan A/S, 2850 Soeborg (DK)
(72) Inventor: WOLFF, Joergen, DK-3460 Birkeroed (DK)
(74) Representative: HOEIBERG A/S
(86) International application number: PCT/DK2002/000898
(87) International publication number: WO 2003/055531

(56) References cited:
- WO-A-95/31955
- WO-A-96/07472
- WO-A-99/44901
- FR-A- 2 759 980
- US-A- 4 522 302
- US-A- 4 891 359
- US-A- 5 798 091
- US-A- 6 045 570

## Description

### FIELD OF THE INVENTION

A process for preparing a powdered haemostatic agent in a containment unit suitable for adding a liquid to the agent and mixing the contents whilst still In the container allows for the sterile use of the haemostatic agent and greater ease of preparation. The haemostatic agent in powder form, such as gelatin, is present in a volume in the containment unit to allow for suitable mixing of any further agents, such as water, saline, or thrombin.

### BACKGROUND OF THE INVENTION

The use of haemostatic agents provides for control of bleeding in surgical procedures. Haemostatic agents supplement pressure, ligature and other conventional methods of controlling capillary, venous, and arterial bleeding.

The present investigators have commercialised a gelatin sponge as a haemostatic agent. A powdered agent some practical advantages in terms of surface area coverage and can be removed by irrigation and suction. Conventional powdered haemostatic agents are unpractical and risk either contamination of the sterile surgical field. The present Invention addresses the risk of compromised sterility and contamination of the haemostatic agent by providing a kit which allows for the sterile and facile preparation of the haemostatic agent.

WO 01/28603 relates to an Injectable formulation for delivery of a composition comprising an osteogenic protein and a haemostatic gelatin foam paste as well as to a method of making a haemostatic gelatin foam paste suitable for injecting osteogenic protein, the method comprising hydration of Gelfoam^{®} powder with glutamic acid buffer.

US 5,394,886 relates to a skin biopsy plug wherein the plug is a porous sponge made from gelatin material, which is implanted into a wound, swells, absorbs blood, and is completely absorbed in the patient. It relates to a combination of the punch (the blade for excising skin) and the plug. The plug used is the commercially available Gelfoam®.

US 5,645,849 claims a haemostatic patch comprising a biodegradable gelatin matrix, a haemostatic-promoting amount of thrombin and epsilon aminocaproic acid.

JP 62221357 discloses a skin ointment for promoting haemostatic effect comprising thermoplastic resin or rubber dissolved in solvent and contains dispersed gelatin powder. The product is an ointment comprising thermoplastic resin or rubber and a fine powder of collagen, gelatin or chitosan.

FR 2679772 relates to particulate material to create an embolism comprising a polymer coated with a haemostatic or thrombonic agent. The haemostatic agent may be a finely divided gelatin powder.

US 6,096,309 relates to a haemostatic composition comprising thrombin and a mixture of non-microfibrillar collagen and microfibrillar collagen in an aqueous medium wherein the microfibrillar collagen has an average fibril diameter of about 3-30 nm.

US 4,515,637 relates to both a method of forming a collagen-thrombin haemostatic composition and to a lysophilized collagen product, comprising collagen and thrombin.

US 6,045,570 relates to a gelatin powder for use as a haemostatic agent and to a biological sealant comprising a gelatin slurry which includes milled gelatin powder. The slurry preferably comprises Gelfoam^{®} powder mixed with a diluent selected from saline and water. The slurry demonstrates superior flow **characteristics in that** it exhibits minimal dilatency and can be easily injected or introduced through catheter lumens, especially small lumens. The product therefore has very fluid characteristics.

GelFoam^{®} is a commercially available product providing powdered gelatin for application to bleeding surfaces as a haemostatic agent. The powdered gelatin is provided in a full glass jar with a metal lid or in a sachet, each of which are to be opened and the contents of which, i.e. the gelatin, are to be poured into a sterile beaker or bowl. Contamination must be avoided during this process and a sterile technique must be employed when adding a sterile saline solution. The problem of dispersion is avoided by initially compressing the powder with gloved fingers into the bottom of the beaker and then kneading it into the desired consistency. The powder is to be used as soon as the jar or sachet is opened and unused portions are discarded. This requires preparation of the haemostatic agent immediately prior to use. Contamination and sterility is not controlled by the product but rather by the user and by the co-ordination of events following preparation of the agent.

Curacell^{®} is a powdered haemostatic agent comprising oxidised cellulose, caboxycellulosum calcium which is applied as dry powder onto a bleeding area.

Avitene^{®} is a microfibullar collagen haemostat "flour" typically applied dry.

### SUMMARY OF THE INVENTION

The invention relates to a process for preparing a haemostatic agent comprising the steps as disclosed in claim 1.

### DESCRIPTION OF THE INVENTION

The risk of compromised sterility and contamination of the haemostatic agent is addressed by the present investigators by providing the process of claim 1 securing sterile and facile preparation of the powdered haemostatic agent. The present method also allows for greater flexibility and ease of preparation of the agent. Surgeons state that the size of the bleeding area and the rate of the bleeding generally determine the consistency and amount of haemostatic agent. During a surgical procedure, these factors can suddenly and dramatically change. The present invention allows for a rapid, sterile and facile preparation of the haemostatic agent. As stated, conventional products typically require the surgical staff to prepare the agent in advance using an additional mixing container.

The haemostatic agent in powder form is gelatin. The gelatin may be derived from an animal or are synthetically made (recombinant). Suitably, the gelatin originates from porcine but may originate from other mammals. The powder is typically sterile

The powder is typically such that 95% of the powder is less than 1000 microns in size, preferably such that 90% of the second volume is less than approximately 700 µm. In a further preferred embodiment, 50% volume is less than approximately 350 µm.

The powder may be prepared from a gelatin sponge cut into pieces that fit into a mill and that will be bulk packaged into sterilization bags and placed in an oven (dry heat), for hardening for three hours. The gelatin plates raw material are typically manually fed into a rotor knife mill with a sieve for final grinding. The particle size is preferably such that not more than 5% (w/w) is retained on a 1 mm mesh.

The containment unit typically comprises at least one opening and at least one closure-unit for closing the at least one opening. In an interesting embodiment, the closure-unit defines a second internal volume suitable for containing a liquid. The liquid may be present in the closure-unit and this liquid can be released into the gelatin by a mechanical or physical action.

The containment unit may comprise of grooves, protrusions or other physical distortions to the otherwise parallel surfaces of the juxtaposed walls of the containment unit so as to ameliorate or facilitate the mixing of a liquid and the haemostatic agent in powder form.

The haemostatic agent in powder form which is gelatin, is suitably present in an amount from about 0.1 to 50 g, preferably from about 0.2 to 20 g, even more preferably from about 0.4 to 10 g, most preferably from about 0.5 to 5 g, such as 0.75, 1, 1.5, 2, 2.5, 3, 3.5, 4, 4.5, and 5 g.

The containment unit is typically substantially rigid with at least one opening which may be closed with an appropriate closure-unit. The containment unit and closure unit are typically comprised of a material independently selected from the group consisting of plastic, glass, metal or rigid or semi-rigid material. In a preferred embodiment, the material is selected from the group consisting of polypropylene, polyethylene, PVC and PET, more preferably polypropylene and polyethylene, most preferably polyethylene. Polyethylene is most suitable for beta irradiation. The substantially rigid polyethylene or polypropylene container having a single opening may be sealed with a threaded, polyethylene or polypropylene closure. The closure-unit suitably comprises a dust-seal closure.

As stated, the containment unit defines a first internal volume; internal volume comprising a haemostatic agent in powder form of a second volume, said second volume being less than 90% of the first volume. The volume difference is, at least in part, to allow for adequate addition of a third volume and mixing of the volumes. The dimension of the containment unit is suitably to facilitate the mixing. Typically, the dimension of the containment unit is also selected so as to allow for facile removal of the volumes onto the patient or other desired location. Thus, the mouth of the containment unit must be suitably broad. Thus, commercially available wide-mouth or large-mouth containers are preferred.

In a particularly suitable embodiment, the haemostatic agent in powder form is present in a volume occupied by about one gram of powder, said second volume of haemostatic agent in powder form is contained within a containment unit of about 50 to 100 cubic centimetres, such as about 60 to 90 cubic centimetres, such as about 70 to 80 cubic centimetres, typically about 75 cubic centimetres.

In the suitable embodiment wherein the haemostatic agent is present in a volume occupied by about two grams of powder, said second volume of haemostatic agent in powder form is contained within a containment unit of about 75 to 200 cubic centimetres, such as about 80 to 180 cubic centimetres, such as about 90 to 170 cubic centimetres, such as about 100 to 160 cubic centimetres. Typically a second volume occupied by 2 g of powder is contained within a first internal volume of 100 to 200 cubic centimetres.

In a suitable embodiment, a liquid for mixing with the haemostatic agent in powder form is in a unit physically separated from the haemostatic agent in powder form.

In a suitable embodiment, the liquid is thrombin. Thus, the medical may further comprise thrombin. The thrombin may be present in the first containment unit or may be in a unit physically separated from the haemostatic agent in powder form or in a second containment unit containing said thrombin.

Thrombin, in a particularly suitable embodiment, may be added in an amount of 1-20 ml per gram of powder, such as 5-15 ml, such as 7-12 ml.

The liquid may alternatively be selected from the group consisting of water and an aqueous solution such as saline. Suitably, the aqueous solution comprises an isotonicity-adjusting agent. The isotonicity adjusting agent may be sodium chloride or known equivalents to sodium chloride such as dextrose, various organic acids and their salts, inorganic salts such as boric acid, citric acid, tartaric acid and phosphoric acids.

The liquid may comprise thrombin and/or a bacteriostatic agent. Typically, the liquid is sterile

In a typical embodiment, the volume of liquid is less than 35% of the volume of haemostatic agent. The volume of liquid is preferably such that, when combined and mixed with the haemostatic agent in powder form, a doughy paste is formed. More suitably, the volume of liquid is less than 33% of the volume of haemostatic agent such as less than 30%, less than 29%, less than 28%, less than 27%, less than 26%, less than 25%, less than 24%, less than 23%, less than 22%, less than 21%, or less than 20%

The mixing of the liquid and the haemostatic agent in powder form is preferably performed by shaking the containment unit, most preferably the mixing is performed when the at least one opening is separated from the external environment by the closure-unit. Shaking may Involve swirling or agitation of any kind.

In a particularly interesting embodiment, the containment unit is surrounded with on outer wrap so that the containment unit is sterile. This will allow the user to remove the outer packaging and transfer the containment unit into the sterile field. The user, in the sterile field, can then add the volume of liquid by opening the containment unit to the external environment, adding or pouring the liquid through the at least one opening of the containment unit.

The medical device preferably further comprises an outer packaging defining a sterile barrier seal for enclosing said containment unit and maintaining sterility of the containment unit and its contents. The outer packaging may be peelable or removed from the outer surface of the containment unit. This containment unit is preferably enclosed in an outer packaging of a flexible, semi-rigid, or rigid plastic and/or metallic film providing a sterile barrier. The outer packaging typically consists of materials selected from the group consisting of Plastic/Aluminium foil/Plastic laminate where the plastic is selected from the group consisting of PET, PE, LLDPE, CPP, PA, PETP, METPET, Tyvek and optionally bonded with an adhesive (Polyurethane or other) or co-extruded. The outer packaging preferably forms a complete barrier to moisture.

The outer packaging may, in a particularly interesting embodiment, be able to endure radiation sterilisation at 3.5 Mrad (Beta) with a bioburden of less than 100 CFU/unit. A particularly interesting embodiment of the outer packaging included a pouch of laminated foil. The laminate may be PET, such as of approximately 12 microns in thickness.

The containment unit is preferably sterile. The containment-unit-facing side of the outer packaging and the containment unit are preferably sterile.

Preferably, the mixing of the liquid and the haemostatic agent in powder form occurs without exposure to an environment external to that of the containment unit, typically without exposure to a non-sterile field.

The relative volume of the haemostatic agent in powder form relative to the internal volume of the containment unit is less than in conventional products so as to be suitable for adding a volume of liquid and for said liquid to be evenly and easily physically dispersed throughout the haemostatic agent in powder form. Moreover, the mixing process, which accelerates the dispersion of the liquid evenly throughout the haemostatic agent in powder form, is facilitated and ameliorated by a low volume of haemostatic agent to volume of the containment unit. Thus, in a particularly preferred embodiment, the volume of haemostatic agent in powder form is less than 85% of the internal volume of the containment unit such as less than 80 % of the first volume, preferably less than 75%, even more preferably less than 70% of the first volume 65% 60% 55%, or 50% of the first volume.

The mixing process occurs within the containment unit and typically without exposure to an environment external to that of the containment unit. That is to say that a physical barrier, such as a closure-unit, typically separates the internal volume, the volume of haemostatic agent in powder form and the volume of liquid from the external environment during the mixing process.

The containment unit is typically rigid, comprising at least one opening and one closure-unit and such that the mixing proceeds without loss of liquid and as described *supra.* The containment unit may be surrounded by a outer wrap/outer packaging se described *supra.*

A further aspect of the invention relates to a process for preparing a haemostatic agent comprising the steps disclosed in claim 1.

Typically, the mixing of haemostatic agent in powder form and liquid proceeds without substantial exposure of said haemostatic agent in powder form and said liquid to an environment external to that the containment unit and without exposure to a non-sterile field. The haemostatic agent in powder form is gelatin.

The process typically involves the containment unit being rigid, and comprising at least one opening and one closure-unit and such that the mixing proceeds without loss of liquid.

The volume of haemostatic agent in powder form is typically less than 85 % of the volume of the containment unit, preferably less than 80%, even more preferably less than 75% of the volume of the containment unit such as 70%, 65%, 60%, 55%, or 50% of this volume.

The sterile containment unit may further comprise an outer packaging defining a sterile barrier seal for enclosing said containment unit, as described supra.

The invention may be used in any array of surgical procedures wherein bleeding or fluid control is preferred, such as In orthopedic procedures, such as laminectomy, total hip replacement and hip revisions, knee surgery, spinal fusion; In cardiothoracic /cardiovascular procedures such as CABGs, valve replacements, aortic surgery, abdominal aortic aneurisms, carotid endarterectomy, and femoral-popliteal bypass, amongst others.

The invention is further defined by the Examples.

### EXAMPLES

### Example 1

### Product and Preparation

Clinical data (Example 2) was obtained on powder prepared from milling sheets of Surgifoam^{®} sponge under a controlled process to produce a product meeting the following specification:

The powder Is a porcine gelatin based powder, off white in colour.
10% volume is less than approximately 90 µm;
50% volume is less than approximately 350 µm;
90% volume is less than approximately 700 µm;
as determined by laser diffraction.

The milled product is collected under slight negative pressure to avoid particulate emission in the area and filled into a primary container, such as by using a hopper and scoop filling operation or by using pharmaceutical cGMP standard auger filling equipment.

The fill will be such that to be less than 75% of the volume of the primary container and checked by electronic scales so as to meet to desired pre-determined weight. A closure will be applied.

The container will then be placed in a foil/film pouch which is sealed with a rotary heat sealer to form a moisture protection poach.

Alternatively, the batched containers will then be transferred to a blister packing room, such as a Multivac blister packing, where each container is packaged into a PETG/PE blister package. The blister packed product is loaded into irradiation boxes and subjected to pre-determined levels of e-beam irradiation.

### Example 2

### Clinical Data

Clinical tests for effectiveness on general surgical procedures as well as cardiovascular and orthopaedic surgical procedures were performed.

### Study Design.

An open label, randomised, controlled, multi-centre, unmasked study was conducted to evaluate safety and effectiveness of two haemostatic agents. The study compared the SURGIFOAM sponge from which the haemostatic powder is prepared to an absorbable gelatin sponge currently legally marketed in the USA. The primary objective was to examine the effectiveness as measured by haemostatis within 10 minutes of application.

### Study Results

Two hundred and eighty one patients were enrolled in to the study and received study treatment. The haemostatis data was collected immediately during the surgery and the patients examined at 2 to 4 weeks and again at 6 to 8 weeks in order to obtain safety data. The effectiveness data is summarised in Table 1.

**Table 1**

| Minutes | Device | General Surgery | Cadiovascular | Orthopaedic | Total |
|---|---|---|---|---|---|
| | | % | % | % | % |
| 3 | Surgifoam | 65.6 | 57.4 | 91.7 | 64.0 |
| | Control | 66.2 | 62.9 | 100 | 66.9 |
| 6 | Surgifoam | 98.4 | 80.9 | 100 | 90.1 |
| | Control | 95.4 | 91.9 | 100 | 94.2 |
| 10 | Surgifoam | 100 | 89.7 | 100 | 95.1 |
| | Control | 95.4 | 96.8 | 100 | 96.4 |

Statistical analysis showed that SURGIFOAM and the control sponge were equivalent In the ability to achieve haemostatis within 10 minutes.

### Example 3

### Evaluation of the Medical Device

### Goal

The purpose of this study was to measure both surgeons' and operating room nurses' perception of the device in terms of control of sterility and ease of preparation.

### Method/Sample

12 interviews were conducted with operating room nurses who use haemostatic agents regularly.

### Summary of Results

The surgeons and the nurses had complete acceptance and preference for a kit as used according to the present invention over conventional products.

### Conventional Practice

Mixing of Conventional Products:
- use of finger in a container separate than the agents container
- use of forceps or pickups or other surgical instruments in a container separate • than the agents container

Disadvantages of Conventional Products:
- possible contamination in transferring product to separate mixing container
- bad smell
- non-sterile packaging
- powder is readily air-borne during opening and transfer process
- non-sterile container risks contamination of sterile field
- lengthy preparation due to requirement of additional bowl
- breathing in of air-borne powder
- wasted product in dispensing to mixing container

A medical device as used according to the present invention was presented to the surgeons and the nurses of the test study. The response was such that the invention favourably addressed most or all of the disadvantaged of conventional products.

### Example 4

### Sealed pouches of laminated foil for containment unit

The pouch must be able to endure radiation sterilisation at 3.5 Mrad (Beta) Bioburden less than 100 CFU/unit. With the Pouch A, suitable containment comprising 1 gram of powder allowed for the storage of the powder and the containment unit as sterile for extended periods of time (typically at least 4 years) without loss in the quality of the materials.

| | | |
|---|---|---|
| Material: | PET | 12 microns |
| | Foil | 8.75 microns |
| | Clear EZ Peel | 50 microns |
| | | |
| Size: | Width | 6.5 inches |
| | Length | 10 inches, |

with a thumbnotch at the chevron end to allow for easy opening.

| | |
|---|---|
| Provider | Perfecseal |

## Claims

1. (67) A process for preparing a haemostatic agent comprising the steps of:
A) removing the outer packaging and transferring the containment unit of the medical device comprising:
i) a containment unit defining an internal volume and being comprised of a material impermeable to a fluid,
wherein the containment unit further comprises at least one opening and at least one closure-unit for closing the at least one opening, and
ii) a sterile haemostatic agent in powder form contained in said containment unit and having a volume of less than 90% of the internal volume of the containment unit;
wherein 95% of said haemostatic agent in powder form has a particle size less than 1000 microns,
wherein said haemostatic agent in powder form is gelatine, and
iii) an outer packaging defining a sterile barrier seal enclosing said containment unit;
wherein the remaining volume of at least 10% of the internal volume is a void volume allowing for the addition of a liquid to the sterile haemostatic agent in powder form through the at least one opening
into a sterile field,
B) Opening the containment unit to the external environment
C) adding a sterile liquid to the containment unit of the medical device,
D) separating the at least one opening of the containment unit of the medical device from the external environment by the closure-unit, and
E) mixing the liquid and the sterile gelatine in powder form contained in the containment unit without substantial exposure of said gelatine and said liquid to an environment external to the containment unit, thereby preparing the haemostatic agent.

## Patentansprüche

1. (67)Verfahren zur Herstellung eines hämostatischen Mittels, umfassend die Schritte:
A) Entfernen der äußeren Verpackung und Überführen der Behältereinheit der medizinischen Vorrichtung, welche:
i) eine Behältereinheit, die ein Innenvolumen definiert und von einem für eine Flüssigkeit undurchlässigen Material umfasst ist,
wobei die Behältereinheit ferner mindestens eine Öffnung und mindestens eine Verschlusseinheit umfasst, um die mindestens eine Öffnung zu verschließen,
ii) ein steriles hämostatisches Mittel in Pulverform, das in der Behältereinheit enthalten ist und ein Volumen von weniger als 90 % des Innenvolumens der Behältereinheit hat;
wobei 95 % des hämostatischen Mittels in Pulverform eine Teilchengröße von weniger als 1000 Mikrometer haben,
wobei das hämostatische Mittel in Pulverform Gelatine ist, und
iii) eine äußere Verpackung, die eine sterile Sperrschichtabdichtung definiert, die die Behältereinheit einschließt;
wobei das restliche Volumen von mindestens 10 % des Innenvolumens ein Hohlraumvolumen ist, das die Zufuhr einer Flüssigkeit zum sterilen hämostatischen Mittel in Pulverform durch die mindestens eine Öffnung erlaubt,
umfasst, in einen sterilen Bereich,
B) Öffnen der Behältereinheit zur äußeren Umgebung,
C) Zuführen einer sterilen Flüssigkeit zur Behältereinheit der medizinischen Vorrichtung,
D) Abtrennen der mindestens einen Öffnung der Behältereinheit der medizinischen Vorrichtung von der äußeren Umgebung durch die Verschlusseinheit, und
E) Mischen der in der Behältereinheit enthaltenen Flüssigkeit und der sterilen Gelatine in Pulverform ohne dass die Gelatine und die Flüssigkeit wesentlich mit einer Umgebung außerhalb der Behältereinheit in Berührung kommen, wodurch das hämostatische Mittel hergestellt wird.

## Revendications

1. Procédé de préparation d'un agent hémostatique comprenant les étapes consistant à :
A) retirer l'emballage externe et transférer l'unité de confinement d'un dispositif médical comprenant :
i) une unité de confinement définissant un volume interne et étant constituée d'un matériau imperméable à un fluide,
l'unité de confinement comprenant en outre au moins une ouverture et au moins une unité de fermeture pour fermer au moins une ouverture, et
ii) un agent hémostatique stérile sous forme de poudre contenue dans ladite unité de confinement et ayant un volume de moins de 90 % du volume interne de l'unité de confinement ;
95 % dudit agent hémostatique sous forme de poudre ayant une taille de particules inférieure à 1000 microns,
ledit agent hémostatique sous forme de poudre étant la gélatine, et
iii) un emballage externe définissant une barrière d'étanchéité stérile renfermant ladite unité de confinement ;
le volume restant d'au moins 10 % du volume interne étant un volume de vide permettant l'addition d'un liquide à l'agent hémostatique stérile sous forme de poudre par au moins une ouverture,
dans un champ stérile,
B) ouvrir l'unité de confinement à l'environnement externe,
C) ajouter un liquide stérile à l'unité de confinement du dispositif médical,
D) séparer au moins une ouverture de l'unité de confinement du dispositif médical de l'environnement externe par l'unité de fermeture, et
E) mélanger le liquide et la gélatine stérile sous forme de poudre contenue dans l'unité de confinement sans exposition substantielle de ladite gélatine et dudit liquide à un environnement externe à l'unité de confinement, ce qui permet de préparer ainsi l'agent hémostatique.
